# EUROPÄISCHE PATENTSCHRIFT

(11) **EP 0 416 408 B1**
(45) Veröffentlichungstag und Bekanntmachung des Hinweises auf die Patenterteilung: **03.08.1994**
(21) Anmeldenummer: 90116323.8
(22) Anmeldetag: 25.08.1990
(51) Int. Cl.: G01M 11/00, A61B 1/06

(54) **Prüfungseinrichtung für Endoskopausrüstungen**
Checking device for endoscope equipment
Dispositif de contrôle pour équipements endoscopiques

(30) Priorität: 06.09.1989 DE 3929562
(43) Veröffentlichungstag der Anmeldung: 13.03.1991
(73) Patentinhaber: Richard Wolf GmbH, 75438 Knittlingen (DE)
(72) Erfinder: Ams, Felix, D-7539 Kämpfelbach (DE); Schäfer, Roland, D-7518 Bretten-Dürrenbüchig (DE)
(74) Vertreter: Wilcken, Thomas, Dipl.-Ing.

(56) Entgegenhaltungen:
- EP-A- 0 061 748
- DE-A- 3 337 454
- DE-A- 3 515 612
- DE-U- 8 815 733
- US-A- 4 556 314
- PATENT ABSTRACTS OF JAPAN, Band 10, Nr. 358 (P-522)(2415), 2. Dezember 1986; & JP - A - 61155833
- REVIEW OF SCIENTIFIC INSTRUMENTS, Band 59, Nr. 6, Juni 1988, Seiten 930-933, New York, NY, US; P. LENZ: "Endoscopic fluorescence detector"
- The Dyonics Autobrite Illuminator Manual, 2 January 1985, 12 Seiten, von Dyonics Inc., Andover, MA, US

## Beschreibung

Die Erfindung geht aus von einem Lichtquellengerät für ein Endoskop gemäß dem Oberbegriff des Patentanspruchs 1. Ein solches Lichtquellengerät ist aus der Firmenschrift der Dyonics Inc., Andover, MA, USA, "The Dyonics Autobrite Illuminator Manual", veröffentlicht am 02.01.1985, 12 Seiten, bekannt. Je eine Skala gibt Auskunft über den Zustand der Lampe und des Lichtleitkabels.

Nachteilig bei diesem Lichtquellengerät ist, daß dessen System bezüglich des Faserbündel-Durchmessers des zur Anwendung gelangenden Lichtleitkabels kalibriert sein muß; bei anderen Faserbündel-Durchmessern ergeben sich Meßungenauigkeiten. Des weiteren werden die gemessenen Helligkeitswerte nur angezeigt und der Anwender muß diese Werte selbst interpretieren, d.h. die Transmission selbst abschätzen, so daß Fehlinterpretationen nicht auszuschließen sind.

Zur Untersuchung schwer zugänglicher Hohlräume, z.B. Körperhöhlen, werden Endoskope eingesetzt. Da die in den Hohlräumen interessierenden Objekte häufig fotografiert oder mit Hilfe von Fernsehkameras auf Monitoren dargestellt werden, werden die benutzten Endoskope mit regelbaren Beleuchtungseinrichtungen betrieben, die für eine optimale Ausleuchtung der betrachteten Gegenstände sorgen sollen. Das von den Lichtquellengeräten bereitgestellte Licht wird dabei mit Lichtleitkabeln zum Endoskop übertragen.

Das Lichtleitkabel einer Endoskopeinrichtung kann beispielsweise durch Bruch einzelner Lichtleitfasern, z.B. infolge einer starken Biegebeanspruchung, durch Alterung oder dergleichen in seiner Funktion beeinträchtigt sein, was dazu führen kann, daß keine optimale Ausleuchtung der mit dem Endoskop betrachteten Objekte mehr möglich ist. Ebenso verringert sich speziell bei Bogenlampen mit zunehmender Betriebsdauer die abgebbare Lichtmenge, so daß auch hierdurch die Ausleuchtung verschlechtert wird. Es ist daher bei Endoskopanwendungen, insbesondere in der Medizin, vorteilhaft, die ordungsgemäße Funktion der Beleuchtungseinrichtung prüfen zu können. Durch eine solche Prüfung wird erreicht, daß eine Fehlfunktion der Beleuchtungseinrichtung und des Lichtleitkabels vor Einführen des Endoskopes in die Körperhöhle festgestellt und daher eine unnötige Beeinträchtigung des Patienten durch Auswechseln eines defekten Lichtleitkabels bzw. einer Lampe mit nicht mehr ausreichender Lichtleistung vermieden werden kann.

Aus der DE-A-35 15 612 ist ein Verfahren zur Überprüfung einer Lichtquelle für Endoskope bekannt. In der dort beschriebenen Ausführungsform werden die Lichtabgabe der Lampe im Dauerlichtbetrieb und im Blitzbetrieb, die Funktion eines Verschlußflügels, die Funktion einer Blende und die Datenübermittlung zwischen Steuereinheit und Kamera geprüft. Nicht geprüft wird die Funktion des Lichtleitkabels, das zum Endoskop führt.

In der DE-C-32 46 290 ist ein Strahlungsenergieüberwachungssystem für eine optische Faser beschrieben, wobei die Pheripherie des Lichtkegels am distalen Ende der optischen Faser zur kontinuierlichen Messung der Strahlungsenergie mittels Thermoelementen herangezogen wird. Dieses System ist so ausgebildet, daß im Infrarotbereich und in dem benachbarten Bereich sichtbaren Lichtes eine ausreichende Empfindlichkeit erhalten und eine thermische Beschädigung der Lichtdetektoranordnung vermieden wird. Bei diesem System wird die insbesondere in einem Handstück, wie es in der Laserchirurgie Verwendung findet, angeordnete optische Faser auf ihre Brauchbarkeit überprüft. Dabei wird der kontinuierlich während der Arbeit mit dem Lasergerät gemessene Energieausgangswert mit einem voreingestellten Bezugswert verglichen. Mit diesem System werden jedoch eventuelle Schwankungen der eingekoppelten Energie nicht separat erfaßt. Man kann also im Falle eines ungenügenden Lichtmengenaustrittes nicht erkennen, welches Element des gesamten Systems für den Defekt verantwortlich ist, also insbesondere nicht entscheiden, ob der Fehler der optischen Faser oder der Lampe zuzuordnen ist.

In der DE-A-34 25 671 ist ein Verfahren und eine Vorrichtung zum sehr genauen Messen der Dämpfung an Lichtwellenleitern unter Berücksichtigung von Änderungen der Sendeleistung beschrieben. Hierzu wird die vom Sender abgestrahlte Lichtleistung erfaßt und in einem Pulsfrequenzmodulationsverfahren die Impulslänge entsprechend der Änderung der Sendeleistung unter Beibehaltung der mittleren Lichtleistung verändert und als kodiertes Signal vom Empfänger erfaßt. Nach Messung der tatsächlichen Empfängerleistung am Empfangsort wird die "Dämpfung" als Differenz zwischen Sendeleistung und Empfängerlichtleistung ermittelt. Die Definition von "Dämpfung" findet sich in DIN 57888, Teil 1, Juni 1984. Der Transmissiongsgrad (Durchlässigkeit) des Lichtwellenleiters, nämlich das Verhältnis von Leistung am Ausgang des Lichtwellenleiters zur Leistung am Eingang des Lichtwellenleiters, läßt sich aus der Dämpfung errechnen. Den bei dem aus der DE-A-34 25 671 bekannten Gerät notwendigen hohen Aufwand wird man aber zur Überprüfung der Funktiongsfähigkeit eines Lichtleitkabels in der Endoskopie nicht betreiben.

Aus der JP-A-61-155 833 ist eine Vorrichtung zur Überprüfung von Lichtleitern bekannt, bei welcher die Lichtmengen am Eingang und Ausgang eines Lichtleiters erfaßt werden und die erfaßten Werte in einer Recheneinheit einer Quotientenbildung unterworfen werden.

Aus der EP-A-0 061 748 ist ein Beleuchtungsgerät für ein Endoskop bekannt, bei dem eine entsprechende Erfassungseinrichtung erkennt, ob ein Lichtleitkabel angeschlossen ist oder nicht, wobei beim Abkoppeln des Lichtleitkabels automatisch über einen Dimmerschaltkreis die Lichtemission der Lichtquelle reduziert oder ganz blockiert wird.

Es ist Aufgabe der Erfindung, ein Lichtquellengerät der einleitend angeführten Art so zu verbessern, daß zusätzlich zur Funktion der Lampe des Gerätes auch die Funktion des verwendeten Lichtleitkabels geprüft werden kann, wobei es nicht auf den Durchmesser des Lichtkeitkabels ankommen soll und wobei die Prüfungsergebnisse erleichtert interpretier bar sind.

Diese Aufgabe wird bei dem Lichtquellengerät gemäß dem Oberbegriff des Patentanspruchs 1 durch die Merkmale nach dem Kennzeichen dieses Patentanspruchs gelöst.

Mit dieser Lösung ist es nun möglich, ohne den Einsatz externer Meßgeräte auf einfache Weise die Helligkeit der Lampe und die Durchlässigkeit des Lichtleitkabels festzustellen, und zwar so, daß an der Anzeige des Gerätes eine einzige Zahlenwertangabe erscheint, die leichter interpretiert werden kann. So ist leichter festzüstellen, ob die Lampe oder das Lichtleitkabel noch die erforderliche Helligkeit zur Verfügung stellen. Beim Überprüfen des Lichtleiterkabels geht die Beschaffenheit der Lampe des Lichtquellengerätes nicht in die Beurteilung der Funktionsfähigkeit des Lichtleiterkabels ein. Des weiteren ist die Prüfung nicht davon abhängig, welchen Durchmesser das betreffende Lichtleitkabel hat. Eine Kalibrierung des Lichtleitkabels ist daher nicht erforderlich.

Um die visuelle Kontrolle der Einzel-Meßergebnisse auf einfache Weise zu ermöglichen, weist das Gerät gemäß dem Anspruch 2 einen Umschalter auf, der beim Unterschreiten des Helligkeitsschwellwertes das durch die erste Erfassungseinrichtung erzeugte Signal für die Helligkeit der Lampe und beim Überschreiten des Helligkeitsschwellwertes ein durch die Rechenschaltung ermitteltes, den Transmissionsgrad des Lichtleitkabels repräsentierendes Signal in Form des Zahlenwertes zur Anzeige bringt.

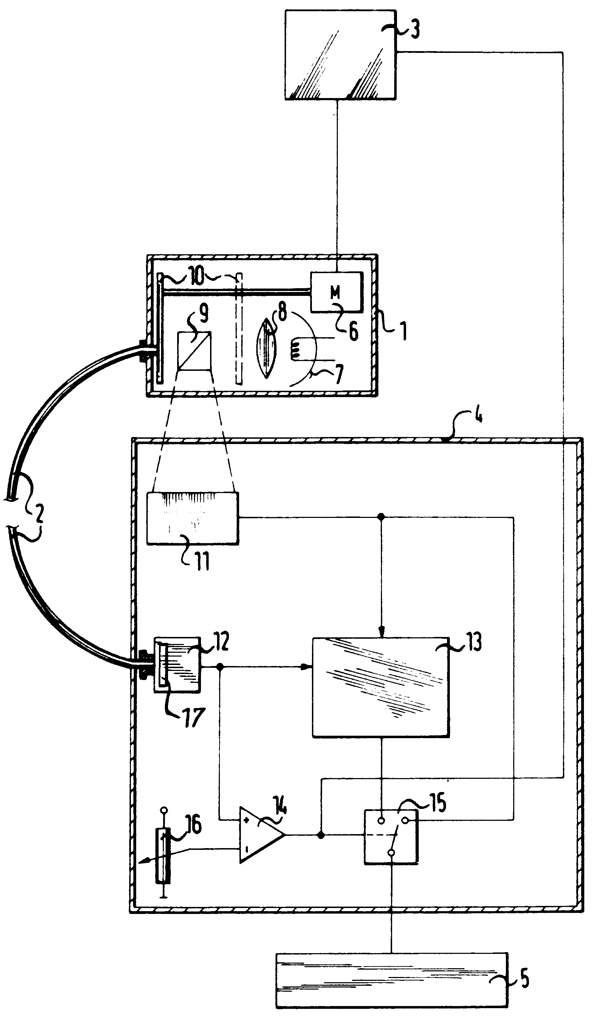

Die Erfindung ist nachfolgend anhand eines in der Zeichnung dargestellten Ausführungsbeispieles näher erläutert.

Das in der einzigen Figur der Zeichnung gezeigte Aüsführungsbeispiel enthält die erfindungsgemäß ausgebildete Anordnung ein Lichtquellengerät 1, das der eintrittseitigen Stirnfläche des Lichtleitkabels 2 fokussiertes und gefiltertes Licht zuführt, eine Blendenregeleinrichtung 3, die an das Lichtquellengerät 1 die zur Lichtmengenregelung notwendigen Steuersignale gibt, eine Transmissionsmeßeinrichtung 4 zur Messung des Transmissionsgrades und eine Anzeige 5 zum Anzeigen des Transmissionsgrades und der Lampenhelligkeit.

In dem Lichtquellengerät 1 sind ein Motor 6, eine Lampe 7, eine Vorrichtung 8 zur Fokussierung und Filterung des von der Lampe 7 abgestrahlten Lichtes, ein Strahlenteiler 9, der einen Teil des Lichtes der Lampe 7 einer Erfassungseinrichtung 11 für die Helligkeit der Lampe 7 zuführt, sowie eine Blende 10 zur Regelung der Lichtmenge angeordnet. Die Anordnung des Strahlenteilers 9 im Lichtweg hat den Vorteil, daß Meßfehler, wie sie beispielsweise bei der Heranziehung von Streulicht zur Messung der Lampenhelligkeit auftreten können, vermieden werden. Über den Motor 6 wird nach Maßgabe der ihm von der Blendenregeleinrichtung 3 zugeführten Signale die Blende 10 geöffnet oder geschlossen, um die Lichtzufuhr in das Lichtleitkabel 2 zu regeln.

Die Transmissionsmeßeinrichtung 4 umfaßt neben der ersten Erfassungseinrichtung 11 weiter eine zweite Erfassungseinrichtung 12 für die an dem distalen Ende des Lichtleitkabels 2 austretende Lichtmenge. Die zweite Erfassungseinrichtung 12 ist mit einer Rechenschaltung 13 und mit einem Komparator 14, der den über einen Regler 16 eingestellten Helligkeitsschwellwert mit dem in der zweiten Erfassungseinrichtung 12 gemessenen Wert vergleicht, verbunden.

Überschreitet das dem Komparator 14 zugeleitete Signal diesen Helligkeitsschwellwert, so wird durch einen Umschalter 15 das durch die Rechenschaltung 13 ermittelte, den Transmissionsgrad repräsentierende Signal der Anzeige 5 zugeführt und dort sichtbar gemacht. Gleichzeitig wird das Komparatorsignal der Blendenregeleinrichtung 3 zugeführt, die wiederum dem Motor 6 des Lichtquellengerätes 1 ein Steuersignal zuführt, so daß demgemäß die Blende 10 auf eine definierte Position (Eichstellung), beispielsweise maximale Blendenöffnung, eingestellt wird.

Der erwähnte Transmissionsgrad ist das Verhältnis aus dem Signal der zweiten Erfassungseinrichtung 12 für die aus dem Lichtleitkabel 2 austretende Lichtmenge und dem Signal der ersten Erfassungseinrichtung 11 für die von der Lampe 7 abgegebene Lichtmenge.

Wird der Helligkeitsschwellwert nicht erreicht oder ist das Lichtleitkabel 2 nicht zur Transmissionsmessung an der Erfassungseinrichtung 12 der Transmissionsmeßeinrichtung 4 angeschlossen, so zeigt die Anzeige 5 die Helligkeit der Lampe 7 z.B. als Zahlenwert an. Dies geschieht dadurch, daß der von der ersten Erfassungseinrichtung 11 aus der vom Strahlenteiler 9 des Lichtquellengerätes 1 auf sie gelenkten Lichtmenge gemessene Helligkeitswert auf die Anzeige 5 gegeben wird.

Wird das Lichtleitkabel 2 zur Überprüfung der Transmission an der zweiten Erfassungseinrichtung 12 angeschlossen und ist die Lampe 7 des Lichtquellengerätes 1 in Betrieb, so wird durch die Erfassungseinrichtung 12 ein Signal erzeugt und sowohl der Rechenschaltung 13 als auch dem Komparator 14 zugeleitet. Im Komparator 14 wird der von der Erfassungseinrichtung 12 übermittelte Wert mit dem durch den Regler 16 eingestellten Helligkeitsschwellwert verglichen. Wird dieser Wert überschritten, so bewirkt das Komparatorausgangssignal die Umschaltung des Umschalters 15, so daß auf der Anzeige 5 der in der Rechenschaltung 13 errechnete Transmissionsgrad des Lichtleitkabels 2 angegeben wird. Der Lichtleiter-Transmissionsgrad wird in der Rechenschaltung 13 ermittelt, indem aus den ihr zugeführten Ausgangssignalen der Lichtmengenerfassungseinrichtungen 11,12 der Quotient gebildet wird. Dadurch ist gewährleistet, daß die Beschaffenheit der Lampe 7 nicht in die Beurteilung der Funktionsfähigkeit des Lichtleitkabels 2 eingeht. Somit kann erkannt werden, ob die Transmission des Lichtleitkabels 2 eine ausreichende Ausleuchtung der zu betrachtenden Objekte zuläßt oder ob das Lichtleitkabel 2 ausgetauscht werden muß.

Um die Ermittlung des Transmissionsgrades vom Faserbündel-Durchmesser des jeweiligen Lichtleitkabels unabhängig zu machen, ist in der Vorrichtung (nichtgezeigt) zur Aufnahme des Lichtleitkabels an der zweiten Erfassungseinrichtung 12 eine Blende 17 so angebracht, daß unabhängig vom tatsächlichen Faserbündel-Querschnitt des Lichtleitkabels stets der gleiche Faserbündel-Teilquerschnitt zur Erfassung der durch das Lichtleitkabel gelangenden Lichtmenge herangezogen wird.

Sowohl die erste Erfassungseinrichtung 11 für die von der Lampe 7 abgegebene Lichtmenge, als auch die Erfassungseinrichtung 12 für die von dem Lichtleitkabel 2 abgegebene Lichtmenge sind Fotoempfänger, z.B. Fotodioden, die ein der empfangenen Lichtmenge entsprechendes elektrisches Signal erzeugen. Die von der Lampe 7 abgegebene Lichtmenge kann auch auf andere Weise, beispielsweise bei stromquellengespeisten Bogenlampen aus der Lampenspannung, ermittelt werden.

Um Lichtquellen geräte verwenden zu können, deren Blende hermetisch schließt, kann die Vorrichtung zum Anschluß des Lichtleitkabels an die zweite Erfassungseinrichtung 12 so gestaltet sein, daß beim Anschluß des Lichtleitkabels 2 die Blende 10 des Lichtquellengerätes 1 etwas geöffnet wird, um der zweiten Erfassungseinrichtung 12 eine zum Überschreiten des Helligkeitsschwellwertes ausreichende Lichtmenge zuzuführen, womit erreicht wird, daß der Motor 6 des Lichtquellengerätes 1 die Blende 10 auf die als Eichstellung gewählte Position einstellt.

Ebenso ist es möglich,die erste Blende 10 zwischen Strahlenteiler 9 und Lampe 7 anzuordnen, wobei dann der berechnete Transmissionsgrad unabhängig von der Stellung der Blende 10 ebenfalls korrekt ermittelt werden kann.

Bei Abkoppeln des Lichtleitkabels 2 von der Erfassungseinrichtung 12 wird durch ein entsprechendes Signal der Blendenregeleinrichtung 3 die erste Blende 10 durch den Motor 6 vollständig geschlossen, wodurch eine mögliche Blendung des Anwenders ausgeschlossen wird.

## Patentansprüche

1. Lichtquellengerät (1) für ein Endoskop, mit einer Lampe (7), einer ersten, steuerbaren Blende (10), einer Regeleinrichtung (3) zum Regeln der an ein an das Gerät (1) anschließbares Lichtleitkabel (2) abgegebenen Lichtmenge, einer ersten Lichterfassungseinrichtung (11) zum Erfassen der an das Lichtleitkabel abgegebenen Lichtmenge und einer zweiten Lichterfassungseinrichtung (12) zum Erfassen der durch das Lichtleitkabel (2) hindurchgegangenen Lichtmenge, dadurch gekennzeichnet, daß die erste Lichterfassungseinrichtung (11) einem in dem Strahlengang der Lampe (7) angeordneten Strahlteiler (9) nachgeordnet ist, daß der zweiten Lichterfassungseinrichtung (12) ein mit einem auf einen Helligkeitsschwellwert einstellbaren Regler (16) zusammenwirkender Komparator (14) nachgeschaltet ist, dessen Ausgangssignal die steuerbare Blende (10) auf eine vorbestimmte Stellung einstellt, daß zur Ermittlung eines den Transmissionsgrad des Lichtleitkabels (2) repräsentierenden Quotienten der ersten (11) und der zweiten (12) Lichterfassungseinrichtung eine Rechenschaltung (13) nachgeschaltet ist, die die von der ersten (11) bzw. zweiten (12) Lichterfassungseinrichtung erfaßten Lichtmengen dividiert, und daß die zweite Lichterfassungseinrichtung (12) eine zweite Blende (17) aufweist, in die das Licht aus dem Lichtleitkabel (2) eintritt.

2. Lichtquellengerät nach Anspruch 1, dadurch gekennzeichnet, daß der Rechenschaltung (13) und dem Komparator (14) ein Umschalter (15) nachgeschaltet ist, der beim Unterschreiten des Helligkeitsschwellwertes das durch die erste Erfassungseinrichtung (11) erzeugte Signal für die Helligkeit der Lampe (7) und beim Überschreiten des Helligkeitsschwellwertes ein durch die Rechenschaltung (13) ermitteltes, den Transmissionsgrad des Lichtleitkabels (2) repräsentierendes Signal in Form des Zahlenwertes der Anzeige (5) zuführt.

## Claims

1. Light source apparatus (1) for an endoscope, having a lamp (7), a first controllable diaphragm (10), a control device (3) for controlling the amount of light emitted at a light-conducting cable (2) which can be connected to the apparatus (1), a first light recording device (11) for recording the amount of light emitted at the light-conducting cable and a second light recording device (12) for recording the amount of light which has passed through the light-conducting cable (2), characterised in that the first light recording device (11) is arranged downstream of a beam splitter (9) arranged in the beam path of the lamp (7), in that a comparator (14) cooperating with a control (16) which can be adjusted to a brightness threshold value is arranged downstream of the second light recording device (12), the output signal of the comparator (14) adjusting the controllable diaphragm (10) to a predetermined position, in that a computing circuit (13) is arranged downstream of the first (11) and the second (12) light recording device to determine a quotient representing the degree of transmission of the light-conducting cable (2), which computing circuit (13) divides the amounts of light recorded by the first (11) or second (12) light recording device, and in that the second light recording device (12) has a second diaphragm (17) into which the light from the light-conducting cable (2) enters.

2. Light source apparatus according to claim 1, characterised in that a switch (15) is arranged downstream of the computing circuit (13) and the comparator (14), which switch (15) supplies the signal produced by the first recording device (11) for the brightness of the lamp (7) when the brightness threshold value is not reached, and supplies a signal representing the degree of transmission of the light-conducting cable (2) determined by the computing circuit (13) when the brightness threshold value is exceeded to the display (5) in the form of a numerical value.

## Revendications

1. Appareil (1) à source de lumière pour un endoscope, comportant une lampe (7), un premier diaphragme commandable (10), un dispositif de régulation (3) pour régler la quantité de lumière envoyée à un câble formant guide de lumière (2), qui peut être raccordé à l'appareil (1), un premier dispositif de détection de lumière (11) servant à détecter la quantité de lumière délivrée dans le câble formant guide de lumière, et un second dispositif de détection de lumière (12) pour détecter la quantité de lumière qui a circulé dans le câble formant guide de lumière (2), caractérisé en ce que le premier dispositif de détection de lumière (11) est disposé en aval d'un diviseur de faisceau (9), disposé dans le trajet du rayonnement de la lampe (7), en ce qu'en aval du second dispositif de détection de lumière (12) est branché un comparateur (14) qui coopère avec un régulateur (16) réglable sur une valeur de seuil de luminosité, et dont le signal de sortie règle le diaphragme commandable (10) sur un réglage prédéterminé, en ce que pour la détermination d'un quotient, qui représente le degré de transmission du câble formant guide de lumière (2), en aval des premier et second dispositifs de détection de lumière (11,12) est branché un circuit de calcul (13), qui divise les quantités de lumière détectée par les premier et second dispositifs de détection de lumière (11,12), et en ce que le second dispositif de détection de lumière (12) possède un second diaphragme (17), dans lequel pénètre la lumière provenant du câble formant guide de lumière (2).

2. Appareil à source de lumière selon la revendication 1, caractérisé en ce qu'en aval du circuit de calcul (13) et du comparateur (14) est branché un commutateur (15), qui, lors du franchissement par valeurs décroissantes de la valeur de seuil de luminosité, délivre sur sa sortie le signal qui est produit par le premier dispositif de détection (11), pour la luminosité de la lampe (7) et, lors du dépassement de la valeur de seuil de luminosité, délivre sur sa sortie un signal déterminé par le circuit de calcul (13) et représentant le degré de transmission du câble formant guide de lumière (2), ces signaux étant sous la forme d'une valeur numérique et étant envoyés au dispositif d'affichage (5).
